Europäisches Patentamt

(19) European Patent Office

Office européen des brevets

(11) EP 0 959 356 B1

(12) **FASCICULE DE BREVET EUROPEEN**

(45) Date de publication et mention
de la délivrance du brevet:
**10.12.2003 Bulletin 2003/50**

(51) Int Cl.$^7$: **G01N 33/96**, G01N 33/86

(21) Numéro de dépôt: **99401218.5**

(22) Date de dépôt: **20.05.1999**

(54) **Compositions utiles dans une méthode de détection d'une résistance à la protéine C activée**

Methode zum Nachweis einer APC-Resistenz und dazu geeignete Zusammensetzungen

Compositions useful in a method of detecting activated protein C resistance

(84) Etats contractants désignés:
**DE ES FR IT SE**

(30) Priorité: **22.05.1998 FR 9806464**

(43) Date de publication de la demande:
**24.11.1999 Bulletin 1999/47**

(73) Titulaire: **Diagnostica Stago**
**92600 Asnières (FR)**

(72) Inventeurs:
• **van Dreden, Patrick**
**94700 Maisons-Alfort (FR)**
• **Martinoli, Jean-Luc**
**94700 Villeneuve-la-Garenne (FR)**

(74) Mandataire: **Warcoin, Jacques**
**Cabinet Régimbeau**
**20, rue de Chazelles**
**75847 Paris cedex 17 (FR)**

(56) Documents cités:
**EP-A- 0 711 839**          **EP-A- 0 761 686**
**EP-A- 0 787 989**          **WO-A-91/01497**
**WO-A-94/17415**

• **M KRAUS, J RÖMISCH: "Factor V Leiden-like behaviour of animal plasma and its use for calibration of activated protein C-dependent assays" BLLOD COAGULATION AND FIBRINOLYSIS, vol. 7, no. 3, avril 1996 (1996-04), pages 295-302, XP002094489**

## Description

L'invention concerne l'utilisation d'un sérum bovin riche en facteur Va pour la préparation de compositions utiles en tant que contrôles pathologiques ou calibrants dans des méthodes d'analyse de la résistance à la protéine C activée (PCa), ainsi que lesdites compositions, leur procédé de préparation et une méthode de détection de la résistance à la protéine C activée.

L'étude des thrombophilies héréditaires a permis de démontrer que la résistance à la PCa (plus communément dénommée selon l'abréviation anglaise APC-R) est principalement associée à des mutations sur le gène codant pour le facteur V. La plus connue, dite "mutation de Leiden", "mutation R506Q", ou "mutation [506]R→[506]Q", se traduit, au niveau de la séquence des acides aminés du facteur V humain, par le remplacement en position 506 d'un résidu Arg en Gln, et prédispose à la thrombose le plus souvent veineuse. Une importante bibliographie existe aujourd'hui sur le sujet. On se réferera notamment aux publications de Dahlbäck B. et al. (1993), Bertina R.M. et al. (1994), Kalafatis M. et al. ( 1994), De Ronde H. et al. (1994), Svensson P.J. et al. (1994) et Dahlbäck et al. (1994).

D'autres anomalies du facteur V peuvent être à l'origine de APC-R. Il se pourrait en particulier que des mutations en position 306 (c'est-à-dire [306]Arg) de la séquence des acides aminés du facteur V et/ou Va (facteur V activé) soient l'une des autres causes possibles de l'APC-R.

Les premières approches de l'APC-R ont été mises en oeuvre (notamment selon un test APTT, KCCT ou analogue) pour apprécier l'existence d'un facteur de risque thrombotique en comparant le temps de céphaline activée, en présence et en l'absence de PCa exogène, d'un plasma témoin avec celui d'un échantillon à tester. L'addition de PCa induit normalement un allongement du temps de coagulation. En effet, la PC sous sa forme activée forme un complexe avec la protéine S et procède ainsi notamment à la dégradation protéolytique du facteur Va. Ceci a pour effet de freiner l'activité thrombotique de la thrombine. De ce fait, la coagulation est retardée. En cas d'anomalie, cet allongement du temps de coagulation se trouve plus réduit que celui observé chez le témoin sain (Mitchell C.A.et al. ( 1987), Amer L. et al. (1990) et Dahlbäck B. et al (1991).

Un kit de dosage est actuellement disponible sur le marché, commercialisé sous la dénomination de "COATEST APC-RESISTANCE" par la société CHROMOGENIX (I.L.). Ce kit met en oeuvre le procédé décrit dans la publication de DAHLBÄCK et al. (1991) précitée et illustré dans WO-A-9310261 et l'article de DAHLBÄCK et al. (1993), pages 1004-1008, précité. Ce procédé comprend le mélange d'un volume de plasma à tester ou de plasma témoin avec un volume de réactif APTT (comprenant des phospholipides + un activateur tel que la silice, le kaolin ou le verre), l'incubation pendant 4 minutes à 37°C, puis l'initiation de la coagulation au moyen d'un volume d'une solution de CaCl$_2$, d'une part, ou d'un même volume d'une solution de CaCl$_2$ additionnée de PCa, d'autre part.

Plusieurs perfectionnements ou alternatives de ce procédé ont été décrits, notamment dans WO-A-9615457, WO-A-9604560 et EP-A-0711838.

Dans tous les procédés décrits, la réponse à la PCa est évaluée en comparant les temps de coagulation obtenus en présence et en l'absence de PCa exogène par des méthodes de coagulation classiques (APTT, PT ou RVVT Xa). Généralement, les résultats sont exprimés sous forme de deux rapports :

■  le rapport standard ou APC-SR avec :

$$APC - SR = \frac{\text{Temps de coagulation en présence de PCa}}{\text{Temps de coagulation en l'absence de PCa}}$$

■  le rapport normalisé ou nAPC-SR avec :

$$nAPC\text{-}SR = \frac{APC - SR \text{ de l'échantillon}}{APC - SR \text{ du plasma témoin}}$$

Toutefois, les résultats obtenus peuvent varier considérablement selon les réactifs et les appareils de mesure utilisés et chaque laboratoire doit définir ses propres normes. De ce fait, les zones de normalité et les valeurs des seuils ("cut-off") présentent également une hétérogénéité. Il est donc important de pouvoir disposer de contrôles et/ou calibrants standardisés pour faciliter l'interprétation des résultats obtenus, en fonction des méthodes utilisées.

Dans le cas des troubles de la coagulation liés à une résistance à la PCa, les contrôles pathologiques ou calibrants utilisés doivent donner des résultats similaires à ceux obtenus à partir du plasma de sujets malades, c'est-à-dire des temps de coagulation en présence de PCa inférieurs à la normale. Toutefois, dans la mesure où cette diminution du temps de coagulation résulte d'une mutation du facteur V, et non d'une absence ou d'une non fonctionnalité de celui-ci, les procédés classiques de préparation de plasmas contrôles (tels que l'adsorption ou la dilution) à partir de plasmas normaux ne peuvent pas être appliqués pour fabriquer des plasmas reflétant cette anomalie.

L'utilisation à une échelle industrielle de plasmas prélevés chez des malades n'étant évidemment pas concevable, il s'est donc avéré nécessaire de trouver des moyens de substitution.

Une solution a été ainsi proposée dans EP 0711839 qui décrit des plasmas pouvant servir de calibrants dans des tests de coagulation évaluant l'activité du système protéine C/protéine S, ou l'APC-R.

Ces plasmas sont obtenus en ajoutant à un plasma humain normal du plasma provenant d'animaux, en particulier du plasma citraté de chien ou préférentiellement de lapin. L'addition de ces plasmas d'animaux produit une diminution du temps de coagulation dans un test dépendant de la PCa proportionnelle à la quantité de plasma non humain ajouté, permettant ainsi de simuler un déficit dans les tests fonctionnels étudiant l'activité du facteur V et/ou du système protéine C/protéine S. EP-0711839 indique également que les plasmas peuvent être plus ou moins appropriés selon l'espèce animale étudiée, sans toutefois fournir une explication précise de cette observation.

L'utilisation de plasmas d'animaux, telle que préconisée dans cette demande offre donc la possiblité d'obtenir facilement des témoins simplifiant l'évaluation de résultats obtenus à partir de tests différents. Toutefois, elle peut présenter certains inconvénients selon les conditions des tests de coagulation effectués.

Ainsi, la simulation d'une APC-R nécessite l'addition d'au moins 5 % de plasma lorsqu'il s'agit du lapin, voire deux à trois fois plus dans le cas d'un autre animal. Cette addition au plasma humain normal peut créer un effet de dilution, susceptible d'interférer dans l'interprétation des résultats. D'autre part, les plasmas animaux utilisés contiennent des taux importants de facteurs II et VII, connus pour être des facteurs d'instabilité (LEWIS J.H., 1996). Des problèmes peuvent donc survenir lors de la conservation de ce type de réactif. Par ailleurs, des problèmes d'approvisionnement peuvent également se poser pour les plasmas préconisés, liés par exemple aux difficultés de prélèvement et faible volume de sang de l'animal (cas du lapin par exemple).

WO91/01497 décrit des contrôles de coagulation stables améliorés mettant en oeuvre le facteur V. Ces contrôles utilisent des plasmas ayant des niveaux élevés de facteur V et de facteur VIII, ainsi qu'au moins environ 2 % en poids d'hydrate de carbone. Cependant, les plasmas décrits ne sont pas des plasmas APC-R.

Les auteurs de la présente invention ont cherché à mettre au point des compositions représentatives d'un plasma contenant un facteur V Leiden et plus précisément un plasma ayant un temps de coagulation en présence de PCa, situé dans l'intervalle défini pour un plasma APC-R. Le but de cette démarche est d'avoir à disposition des plasmas simulant le comportement de plasmas APC-R et s'en servir comme contrôles pathologiques quand le plasma à tester est un plasma APC-R ou fortement soupçonné de l'être.

Deux approches sont théoriquement possibles pour la fabrication d'un tel plasma "APC-R like".

La première consiste à induire une inhibition de la PCa à l'aide de composés spécifiques, créant ainsi une situation dans laquelle le facteur Va n'est plus inhibé.

La seconde approche consiste à induire une saturation de la PCa c'est-à-dire une situation dans laquelle le facteur Va se trouve présent dans des quantités telles que la PCa n'est capable d'en cliver qu'une partie. L'excès de facteur Va restant actif peut alors induire un temps de coagulation identique à celui d'un plasma APC-R.

Les auteurs de la présente invention ont adopté la seconde approche. Ceux-ci se sont donc efforcés de sélectionner une source de facteur Va non humaine qui puisse être ajoutée à un plasma normal de façon à simuler un plasma APC-R, sans que cela n'induise une trop grande dilution ou une instabilité de ce plasma, ni des interférences liées à l'apport concomitant d'autres facteurs.

Les auteurs de la présente invention ont pu observer que l'addition de très faibles quantités de sérum de bovin, riche en facteur Va à du plasma humain normal induisait une diminution du temps de coagulation en présence de PCa. Ceux-ci ont ainsi obtenu des plasmas contrôles pathologiques en sélectionnant, à partir de gammes de dilution de sérum de bovin riche en facteur Va dans des plasmas humains normaux, les échantillons présentant un temps de coagulation situé dans l'intervalle de celui d'un plasma APC-R. Par ailleurs, la diminution du temps de coagulation du plasma étant en fonction de la quantité de sérum ajoutée jusqu'à inhibition totale de l'action de la PCa (seuil correspondant au temps de coagulation minimum), les travaux des inventeurs permettent également de préparer des compositions utilisables en tant que calibrants pour ce type de tests.

Outre les avantages énoncés ci-dessus (faible dilution du plasma, apport négligeable d'autres facteurs de la coagulation et de facteurs interférents dans les réactions), l'utilisation de sérums animaux riches en facteur Va présente un autre intérêt en matière de spécificité. Il a en effet été montré que le facteur V augmentait la dégradation protéolytique du facteur VIII par la PCa, même en l'absence de protéine S, alors que le facteur Va n'avait pas cette action de cofacteur (VARADI K. et al., 1995). Or le facteur V se présente sous sa forme activée dans le sérum, alors qu'il est sous sa forme non activée dans le plasma. L'addition d'un sérum présente donc, par rapport à celle d'un plasma, une plus grande spécificité pour l'action de la PCa sur le facteur Va et est ainsi particulièrement avantageuse pour des tests de détection de l'APC-R.

La présente invention a donc pour objet l'utilisation d'un sérum de bovin riche en facteur Va pour la fabrication de compositions utiles en tant que contrôles pathologiques ou calibrants dans une méthode de détection d'une résistance à la protéine C activée.

Elle vise également des compositions utiles en tant que contrôles pathologiques ou calibrants pour une méthode de détection d'une résistance à la PCa comprenant du sérum de bovin riche en facteur Va et du plasma normal.

L'invention a en outre pour objet, un procédé de préparation de compositions utiles en tant que contrôles pathologiques ou calibrants dans une méthode de détection d'une résistance à la PCa comprenant du plasma normal enrichi en facteur Va bovin.

Par "plasma normal", on entend un plasma normal ou un pool de plasmas normaux.

L'invention comprend enfin une méthode in vitro de détection d'une résistance à la PCa et les kits pour leur mise en oeuvre comprenant, à tire de contrôle pathologique ou calibrant, une composition telle que définie ci-dessus.

Selon un premier aspect, la présente invention a pour objet l'utilisation d'un sérum de bovin riche en facteur Va pour les applications visées ci-dessus.

Ainsi, dans le cadre de la présente invention, par "sérum de bovin", on entend un sérum ou un pool de sérums d'origine bovine.

En effet, par rapport au plasma humain considéré comme contenant 100 % de facteur V, on mesure chez le bovin 500 % de ce facteur dans le plasma, et jusqu'à 2000 à 3500 % de facteur V sous sa forme activée (facteur Va) dans le sérum (ces quantités sont exprimées par rapport à une référence constituée par un pool de plasmas humains de donneurs sains, dans lequel le taux de facteur V est supposé être de 100 % ou 1 U/ml, Karges H.E. et al., *(1994))*.

Un sérum de bovin fortement concentré en facteur Va peut ainsi être utilisé en des quantités beaucoup plus faibles que celles nécessaires pour les plasmas, ce qui permet d'éviter des problèmes de dilution du plasma normal dans les compositions selon l'invention.

Préférentiellement, on utilise un pool de sérum contenant plus de 20 U/ml (2000 %) de facteur Va, et, plus préférentiellement, plus de 22 U/ml (2200 %).

De telles quantités en facteur Va dans le sérum permettent de l'utiliser dans des gammes de dilutions finales comprises entre 1/300 et 1/1200, et préférentiellement entre 1/400 et 1/1000.

Avantageusement, le sérum de bovin utilisé est traité de façon à éliminer ou éviter les effets des facteurs susceptibles d'être à l'origine d'une instabilité ou d'une interférence. Ces facteurs sont notamment des facteurs dépendants de la vitamine K, tels les facteurs II, VII, IX, X, Protéine S, Protéine C, Protéine Z. On élimine également les traces de fibrine.

Ces facteurs peuvent être éliminés en réalisant une adsorbtion du sérum sur du sulfate de baryum, tel que décrit par SOULIER J.P., *(1975)*.

Selon un autre aspect, la présente invention vise des compositions utiles en tant que contrôles pathologiques ou calibrants dans une méthode de détection d'une résistance à la PCa, comprenant du plasma normal et du sérum de bovin riche en facteur Va. Préférentiellement, le plasma normal est du plasma humain, et plus préférentiellement du plasma humain citraté.

Dans une variante avantageuse, lesdites compositions comprennent du sérum de bovin, contenant plus de 20 U/ml (2000 %) de facteur Va et préférentiellement plus de 22 U/ml (2200 %).Le sérum est présent dans les compositions à des dilutions allant du 1/300è au 1/1200è et de préférence du 1/400è au 1/1000è.

Le pool de sérum est de préférence traité comme décrit ci-dessus, par adsorption sur du sulfate de baryum et de la bentonite, ou autre absorbant.

Les compositions selon l'invention peuvent avantageusement se présenter sous forme lyophilisée.

La présente invention comprend également un procédé de préparation desdites compositions. Celles-ci consistent à effectuer une gamme de dilutions d'un pool de sérum de bovin riche en facteur Va, préférentiellement du sérum de bovin contenant plus de 20 U/ml (2000 %) de facteur Va et, plus préférentiellement, plus de 22 U/ml (2200 %), dans un plasma normal ou un pool de plasmas normaux, de préférence humains, et à sélectionner les échantillons pour lesquels le temps de coagulation en présence de PCa se situe dans l'intervalle défini pour le temps de coagulation en présence de PCa d'un plasma APC-R. Un protocole de préparation détaillé des compositions selon l'invention est décrit ci-dessous.

**1. Matières premières :**

- Plasma humain
- Acide chlorhydrique R.P
- Sodium hydroxyde R.P
- Sérum de boeuf adsorbé congelé

**2. Fabrication :**

2.1. Préparation du sérum :

Le sérum est préparé à partir de plasma de bovin coagulé par un mélange de thrombine, phospholipides, $Ca^{2+}$.

- le sérum de boeuf congelé et préalablement adsorbé à 37°C pendant 30 minutes sur du sulfate de Baryum 10 % P/V (2 fois) est décongelé,
- le sérum est adsorbé sur de la bentonite (5‰) pendant 20 minutes, et
- le surnageant obtenu après centrifugation est filtré.

2.2. Sélections des compositions :

- une gamme de dilutions du sérum est préparée dans un pool de plasma humains normaux pour obtenir des dilutions finales du sérum comprises entre 1/300 et 1/1200,

- une agitation est effectuée pendant 5 minutes à température ambiante,

- les échantillons préparés sont testés selon la méthode "STA-Staclot APC-R" telle que décrite dans *"A New Chronometric Assay based on the Va Dependent procoagulant activity of a snake venom for the detection of Factor V Leiden", XVIth Congress of the ISTH - Florence (Italy), 6-12 June 1997 ou dans « Automated detection of the Factor V Leiden using a new screening test : STA Staclot-APC-R »*, 15th International Congress on Thrombosis-Antalya (Turkey) 15-21 October 1998, par rapport à un pool de références (sur STA),

- l'échantillon pour lequel le temps de coagulation en présence de PCa est compris entre 60 et 90 secondes, correspondant au rapport normalisé d'un plasma APC-R compris entre 0,40 et 0,65 est sélectionné.

2.3. Une lyophilisation standard est effectuée pendant 48 heures

L'utilisation des compositions selon l'invention dans une méthode de détection d'une résistance à la PCa in vitro permet de vérifier la qualité du dosage réalisé.

L'invention a donc également pour objet une méthode de détection d'une résistance à la PCa dans un plasma à tester qui consiste à comparer le temps de coagulation mesuré pour ledit plasma avec le temps de coagulation mesuré pour des compositions contrôles conformes à l'invention présentant chacune des dilutions différentes du sérum de bovin riche en facteur Va. Le temps de coagulation mesuré pour la composition conforme à l'invention le plus proche de, voire identique à celui mesuré pour le plasma à tester sera révélateur du niveau de résistance à la PCa dudit plasma car on connaît exactement la quantité de facteur Va ajoutée à ladite composition contrôle.

La méthode de coagulation utilisée peut être chronométrique ou chromogénique. Il s'agit préférentiellement d'une méthode chronométrique basée sur la mesure du temps de coagulation de l'échantillon à étudier, additionné d'un plasma déficient en facteur V, d'un venin (facteur V dépendant) et de Ca $Cl_2$ avec ou sans PCa. Les résultats pourront être exprimés sous forme de rapport normalisé, ou préférentiellement, en temps ou DO.

Les compositions de l'invention peuvent donc avantageusement entrer dans la constitution d'un kit de diagnostic d'une résistance à la protéine C activée, à titre de contrôle pathologique. Elle se présenteront préférentiellement sous forme lyophilisée pour leur conservation, et seront reconstituées au moment de l'emploi avec de l'eau distillée.

La présente invention a également pour objet un tel kit de diagnostic.

L'invention sera mieux comprise à la lumière des exemples ci-après qui ne sont donnés qu'à titre purement illustratif.

**EXEMPLES :**

Dans tous les tableaux ci-après, "-PCa" signifie qu'aucun ajout de PCa n'a été effectué dans le test, "+PCa" signifie que de la PCa exogène a été ajoutée au test.

**Exemple 1 : Préparation d'un calibrant.**

La détermination du temps de coagulation est réalisée sur un automate STA® par un dosage chronométrique selon la méthode STA-STACLOT®-APC®-R citée précédemment.

Le plasma humain normal est du plasma citraté obtenu à partir d'un pool de 30 plasmas normaux.

Le pool de sérum animal utilisé est du sérum de bovin contenant plus de 22 U/ml (2200 %) de facteur Va.

Les temps de coagulation en fonction de la dilution du sérum dans le plasma sont reportés dans le tableau I ci-dessous.

Les temps de coagulation (exprimés en secondes) diminuent avec les dilutions finales du sérum dans le plasma.

L'utilisation d'un sérum selon l'invention permet donc d'obtenir des compositions utiles en tant que calibrants.

Tableau I :

| Dilution finale du sérum | 1/100 | 1/200 | 1/300 | 1/400 | 1/500 | 1/700 | 1/1000 | 0 |
|---|---|---|---|---|---|---|---|---|
| Temps de coagulation | 24,5 | 38,8 | 47,8 | 52,7 | 57,3 | 61,2 | 77,4 | 180 |

## Exemple 2 : Comparaison des résultats obtenus avec la méthode "Coatest APC-résistance" (CHROMOGENIX) et la méthode STA-STACLOT APC-R.

Dans le tableau II sont reportés des résultats obtenus avec un exemple de composition selon l'invention utilisée en tant que contrôle pathologique dans la méthode "COATEST APC-Résistance" et dans la méthode STA®-STACLOT®-APC-R citée précédemment.

Le tableau II indique que les compositions de l'invention peuvent avantageusement être utilisées dans un test de détection de la résistance à la PCa.

### Tableau II :

| | Composition selon l'invention | | | Pool de plasmas humains normaux | | |
|---|---|---|---|---|---|---|
| | Temps de coagulation | | Rapport standard | Temps de coagulation | | Rapport standard |
| | - PCa | + PCa | (APC-SR) | - PC | + PCa | (APC-SR) |
| Méthode COATEST | 39,6 | 61,3 | 1,54 | 57,8 | 218,9 | 3,78 |
| Méthode STA-STACLOT APCR | 38,5 | 67,2 | 1,74 | 50,7 | 177,8 | 3,51 |

## Exemple 3 : Reproductibilité intra-essai.

Afin d'estimer la reproductibilité intra-essais des Contrôles Pathologiques sur STA, on a, à partir de trois compositions contrôles pathologiques, effectué 21 déterminations avec le même kit de réactifs, STA® STACLOT® APC-R. Les résultats concernant le coefficient de variation (CV) sont donnés dans le tableau III ci-dessous. Ils indiquent que le CV du rapport normalisé nSR -APC-R ou du temps en présence de PCa est inférieur à 5 %.

Tableau III :

| | CRT P : 7349A1D | | | | CRT P : 8005A2D | | | | CRT P : 8005B3D | | | |
|---|---|---|---|---|---|---|---|---|---|---|---|---|
| | -PCa | +PCa | APC-SR | n-APC-SR | -PCa | +PCa | APC-SR | n-APC-SR | -PCa | +PCa | APC-SR | n-APC-SR |
| Pool 08/97 | 37,2 | 158,4 | 4,26 | | | | | | | | | |
| | 40,3 | 155,0 | 3,87 | | | | | | | | | |
| 1 | 40,1 | 80,9 | 2,02 | 0,50 | 37,5 | 67,8 | 1,81 | 0,44 | 38,4 | 68,4 | 1,78 | 0,44 |
| 2 | 40,8 | 80,4 | 1,97 | 0,48 | 38,6 | 67,6 | 1,75 | 0,43 | 39,0 | 70,8 | 1,82 | 0,45 |
| 3 | 40,2 | 78,5 | 1,95 | 0,48 | 39,0 | 67,4 | 1,73 | 0,43 | 39,4 | 69,9 | 1,77 | 0,44 |
| 4 | 41,7 | 79,5 | 1,91 | 0,47 | 38,0 | 70,1 | 1,84 | 0,45 | 39,2 | 69,7 | 1,78 | 0,44 |
| 5 | 40,4 | 82,3 | 2,04 | 0,50 | 39,0 | 68,6 | 1,76 | 0,43 | 39,5 | 68,3 | 1,73 | 0,43 |
| 6 | 40,2 | 79,7 | 1,98 | 0,49 | 38,4 | 67,2 | 1,75 | 0,43 | 38,6 | 69,2 | 1,79 | 0,44 |
| 7 | 40,4 | 78,4 | 1,94 | 0,48 | 38,4 | 67,7 | 1,76 | 0,43 | 38,9 | 68,9 | 1,77 | 0,44 |
| 8 | 39,5 | 77,4 | 1,96 | 0,48 | 38,5 | 67,8 | 1,76 | 0,43 | 38,8 | 71,0 | 1,83 | 0,45 |
| 9 | 40,1 | 80,0 | 2,00 | 0,49 | 38,3 | 69,1 | 1,80 | 0,44 | 38,4 | 69,6 | 1,81 | 0,45 |
| 10 | 40,8 | 78,3 | 1,92 | 0,47 | 38,6 | 68,2 | 1,77 | 0,43 | 38,6 | 70,1 | 1,82 | 0,45 |
| 11 | 41,3 | 80,7 | 1,95 | 0,48 | 38,7 | 67,7 | 1,75 | 0,43 | 38,6 | 69,7 | 1,81 | 0,44 |
| 12 | 40,5 | 79,1 | 1,95 | 0,48 | 38,3 | 68,0 | 1,78 | 0,44 | 39,0 | 68,3 | 1,75 | 0,43 |
| 13 | 40,0 | 79,0 | 1,98 | 0,49 | 38,6 | 68,1 | 1,76 | 0,43 | 39,1 | 69,9 | 1,79 | 0,44 |
| 14 | 40,6 | 80,3 | 1,98 | 0,49 | 38,6 | 68,2 | 1,77 | 0,43 | 39,1 | 68,3 | 1,75 | 0,43 |
| 15 | 41,0 | 77,6 | 1,89 | 0,47 | 39,0 | 69,5 | 1,78 | 0,44 | 39,3 | 68,7 | 1,75 | 0,43 |
| 16 | 40,3 | 78,7 | 1,95 | 0,48 | 38,2 | 67,9 | 1,78 | 0,44 | 38,9 | 67,8 | 1,74 | 0,43 |
| 17 | 41,2 | 80,8 | 1,96 | 0,48 | 38,8 | 69,0 | 1,78 | 0,44 | 39,6 | 69,1 | 1,74 | 0,43 |
| 18 | 39,8 | 78,0 | 1,96 | 0,48 | 38,9 | 69,0 | 1,77 | 0,44 | 38,9 | 68,6 | 1,76 | 0,43 |
| 19 | 40,0 | 80,8 | 2,02 | 0,50 | 38,7 | 68,4 | 1,77 | 0,43 | 39,0 | 67,8 | 1,74 | 0,43 |
| 20 | 41,3 | 80,2 | 1,94 | 0,48 | 38,1 | 69,2 | 1,82 | 0,45 | 38,6 | 69,2 | 1,79 | 0,44 |
| 21 | 40,7 | 79,3 | 1,95 | 0,48 | 38,8 | 68,0 | 1,75 | 0,43 | 38,8 | 68,2 | 1,76 | 0,43 |
| MOY | 40,52 | 79,52 | 1,96 | 0,48 | 38,52 | 68,31 | 1,77 | 0,44 | 38,94 | 69,12 | 1,78 | 0,44 |
| SD | 0,56 | 1,26 | 0,04 | 0,01 | 0,37 | 0,75 | 0,03 | 0,01 | 0,34 | 0,92 | 0,03 | 0,01 |
| %CV | 1,38 | 1,59 | 1,81 | 1,81 | 0,97 | 1,10 | 1,49 | 1,49 | 0,87 | 1,33 | 1,66 | 1,66 |

## Exemple 4 : Reproductibilité par lot.

On a ici testé sur STA avec un même lot de réactifs (STA® STACLOT® APC-R : 010), 3 lots de compositions contrôles pathologiques. Chaque plasma est testé 2 fois dans une même série (sur 1 flacon). 15 séries indépendantes (15 flacons du même lot) ont été réalisées.

Le tableau IV ci-après montre que le coefficient de variation (CV) des temps avec PCa est inférieur à 5 %.

**Tableau IV** :

| Flacons | CRT P : 7349A1D | | | | CRT P : 8005A2D | | | |
|---|---|---|---|---|---|---|---|---|
| | -PCa | +PCa | APC-SR | n-APC-SR | PCa | +PCa | APC-SR | n-APC-SR |
| 1 | 42,2 | 83,2 | 1,97 | 0,48 | 39,0 | 68,7 | 1,76 | 0,43 |
| | 42,2 | 83,4 | 1,98 | 0,48 | 39,1 | 70,1 | 1,79 | 0,44 |
| 2 | 41,9 | 83,6 | 2,00 | 0,49 | 39,0 | 68,7 | 1,76 | 0,43 |
| | 42,5 | 82,0 | 1,93 | 0,47 | 39,3 | 68,5 | 1,74 | 0,43 |
| 3 | 41,3 | 82,2 | 1,99 | 0,49 | 39,0 | 68,3 | 1,75 | 0,43 |
| | 42,2 | 81,4 | 1,93 | 0,47 | 39,2 | 68,1 | 1,74 | 0,42 |
| 4 | 42,0 | 83,3 | 1,98 | 0,48 | 40,0 | 68,5 | 1,71 | 0,42 |
| | 41,6 | 82,4 | 1,98 | 0,48 | 39,7 | 68,7 | 1,73 | 0,42 |
| 5 | 42,1 | 81,8 | 1,94 | 0,47 | 39,0 | 68,3 | 1,75 | 0,43 |
| | 41,8 | 79,8 | 1,91 | 0,47 | 39,2 | 69,6 | 1,78 | 0,43 |
| 6 | 42,2 | 82,4 | 1,95 | 0,48 | 39,3 | 67,7 | 1,72 | 0,42 |
| | 41,7 | 81,8 | 1,96 | 0,48 | 39,0 | 69,8 | 1,79 | 0,44 |
| 7 | 41,1 | 80,7 | 1,96 | 0,48 | 39,0 | 68,2 | 1,75 | 0,43 |
| | 41,4 | 80,1 | 1,93 | 0,47 | 39,0 | 68,6 | 1,76 | 0,43 |
| 8 | 41,8 | 82,0 | 1,96 | 0,48 | 38,4 | 68,5 | 1,78 | 0,44 |
| | 41,5 | 81,2 | 1,96 | 0,48 | 39,0 | 68,4 | 1,75 | 0,43 |
| 9 | 41,3 | 81,1 | 1,96 | 0,48 | 39,8 | 67,9 | 1,71 | 0,42 |
| | 41,4 | 80,9 | 1,95 | 0,48 | 40,1 | 70,1 | 1,75 | 0,43 |
| 10 | 40,6 | 80,6 | 1,99 | 0,48 | 39,2 | 67,9 | 1,73 | 0,42 |
| | 41,3 | 81,9 | 1,98 | 0,48 | 39,1 | 67,7 | 1,73 | 0,42 |
| 11 | 40,6 | 78,9 | 1,94 | 0,47 | 39,4 | 69,1 | 1,75 | 0,43 |
| | 41,1 | 82,6 | 2,01 | 0,49 | 40,1 | 67,8 | 1,69 | 0,41 |
| 12 | 40,0 | 79,6 | 1,99 | 0,49 | 40,4 | 67,9 | 1,68 | 0,41 |
| | 41,0 | 79,0 | 1,93 | 0,47 | 40,2 | 68,4 | 1,70 | 0,42 |
| 13 | 39,4 | 81,0 | 2,06 | 0,50 | 39,4 | 68,9 | 1,75 | 0,43 |
| | 39,9 | 82,0 | 2,06 | 0,50 | 39,9 | 67,1 | 1,68 | 0,41 |
| 14 | 39,3 | 81,2 | 2,07 | 0,50 | 39,3 | 68,9 | 1,75 | 0,43 |
| | 39,4 | 79,9 | 2,03 | 0,50 | 39,4 | 68,4 | 1,74 | 0,42 |
| 15 | 38,7 | 81,6 | 2,11 | 0,51 | 38,7 | 68,1 | 1,76 | 0,43 |
| | 38,7 | 81,4 | 2,10 | 0,51 | 38,7 | 68,9 | 1,78 | 0,43 |
| MOY | 41,1 | 81,4 | 1,98 | 0,48 | 39,3 | 68,5 | 1,74 | 0,43 |
| SD | 1,02 | 1,27 | 0,05 | 0,01 | 0,48 | 0,71 | 0,03 | 0,01 |
| % CV | 2,48 | 1,55 | 2,33 | 2,33 | 1,23 | 1,04 | 1,69 | 1,69 |

## Tableau IV (suite):

| Flacons | CRT P : 8005B3D | | | |
|---|---|---|---|---|
| | -PCa | +PCa | APC-SR | n-APC-SR |
| 1 | 39,2 | 69,4 | 1,77 | 0,43 |
| | 40,0 | 69,0 | 1,73 | 0,42 |
| 2 | 39,4 | 69,6 | 1,77 | 0,43 |
| | 39,6 | 68,9 | 1,74 | 0,42 |
| 3 | 40,2 | 68,5 | 1,70 | 0,42 |
| | 39,5 | 68,5 | 1,73 | 0,42 |
| 4 | 39,7 | 69,8 | 1,76 | 0,43 |
| | 39,7 | 70,0 | 1,76 | 0,43 |
| 5 | 39,5 | 72,8 | 1,84 | 0,45 |
| | 39,8 | 69,7 | 1,75 | 0,43 |
| 6 | 40,7 | 69,8 | 1,71 | 0,42 |
| | 39,8 | 71,0 | 1,78 | 0,44 |
| 7 | 39,3 | 69,9 | 1,78 | 0,43 |
| | 39,3 | 69,0 | 1,76 | 0,43 |
| 8 | 39,4 | 68,6 | 1,74 | 0,43 |
| | 39,5 | 68,8 | 1,74 | 0,43 |
| 9 | 39,3 | 68,2 | 1,74 | 0,42 |
| | 39,3 | 67,9 | 1,73 | 0,42 |
| 10 | 39,4 | 68,6 | 1,74 | 0,43 |
| | 39,2 | 69,2 | 1,77 | 0,43 |
| 11 | 39,2 | 69,5 | 1,77 | 0,43 |
| | 39,7 | 68,0 | 1,71 | 0,42 |
| 12 | 39,3 | 67,5 | 1,72 | 0,42 |
| | 40,2 | 68,4 | 1,70 | 0,42 |
| 13 | 39,7 | 66,4 | 1,67 | 0,41 |
| | 40,1 | 64,6 | 1,61 | 0,39 |
| 14 | 39,6 | 67,0 | 1,69 | 0,41 |
| | 39,4 | 66,5 | 1,69 | 0,41 |
| 15 | 39,0 | 65,7 | 1,68 | 0,41 |
| | 39,7 | 67,0 | 1,69 | 0,41 |
| MOY | 39,6 | 68,6 | 1,73 | 0,42 |
| SD | 0,37 | 1,61 | 0,04 | 0,01 |
| % CV | 0,95 | 2,35 | 2,49 | 2,49 |

### Exemple 5 : Reproductibilité jour à jour.

Dans le but d'estimer la reproductibilité jour à jour, un même lot de Contrôle Pathologique a été testé sur 10 jours avec le même lot de réactifs (STA® Staclot® APC-R 010) sur le même STA. Les rapports normalisés découlent d'un dosage journalier du même pool aliquoté.

Le tableau V ci-après montre que le CV des rapports ou des temps en présence de PCa jour à jour est inférieur à 5 %.

Tableau V :

| Jours | CRT P : 7349 AID | | | | CRT P : 8005A2D | | | | CRT P : 8005B3D | | | |
|---|---|---|---|---|---|---|---|---|---|---|---|---|
| | -PCa | +PCa | APC -SR | n-APC -SR | -PCa | +PCa | APC -SR | n-APC -SR | -PCa | +PCa | APC -SR | n-APC -SR |
| Pool 08/97 | 40,1 | 162,4 | 4,05 | | | | | | | | | |
| 1 | 40,9 | 78,9 | 1,93 | 0,48 | 39,1 | 68,0 | 1,74 | 0,43 | 39,7 | 69,9 | 1,76 | 0,43 |
| 2 | 40,8 | 78,4 | 1,92 | 0,47 | 39,7 | 69,4 | 1,75 | 0,43 | 39,5 | 69,2 | 1,75 | 0,43 |
| 3 | 41,8 | 77,9 | 1,86 | 0,46 | 39,3 | 66,3 | 1,69 | 0,42 | 39,7 | 67,4 | 1,70 | 0,42 |
| 4 | 41,9 | 78,5 | 1,87 | 0,46 | 39,3 | 65,8 | 1,67 | 0,41 | 39,6 | 68,7 | 1,73 | 0,43 |
| 5 | 41,7 | 83,2 | 2,00 | 0,49 | 39,0 | 68,4 | 1,75 | 0,43 | 39,7 | 70,5 | 1,78 | 0,44 |
| 6 | 42,2 | 82,4 | 1,95 | 0,48 | 38,8 | 67,8 | 1,75 | 0,43 | 39,6 | 69,4 | 1,75 | 0,43 |
| 7 | 40,7 | 84,5 | 2,08 | 0,51 | 39,4 | 70,2 | 1,78 | 0,44 | 40,3 | 72,3 | 1,79 | 0,44 |
| 8 | 42,2 | 84,9 | 2,01 | 0,50 | 39,6 | 69,3 | 1,75 | 0,43 | 40,2 | 72,2 | 1,80 | 0,44 |
| 9 | 40,8 | 81,1 | 1,99 | 0,49 | 38,6 | 69,5 | 1,8 | 0,44 | 38,9 | 68,9 | 1,77 | 0,44 |
| 10 | 41,5 | 80,6 | 1,94 | 0,48 | 39,1 | 69,6 | 1,78 | 0,44 | 38,9 | 68,3 | 1,76 | 0,43 |
| MOY | 41,45 | 81,04 | 1,96 | 0,48 | 39,19 | 68,43 | 1,75 | 0,43 | 39,61 | 69,68 | 1,76 | 0,43 |
| SD | 0,6 | 2,61 | 0,06 | 0,02 | 0,34 | 1,47 | 0,04 | 0,01 | 0,46 | 1,60 | 0,03 | 0,01 |
| %CV | 1,44 | 3,22 | 3,30 | 3,30 | 0,87 | 2,14 | 2,27 | 2,27 | 1,15 | 2,29 | 1,64 | 1,64 |

**Exemple 6 : Recouvrement lot à lot.**

3 Contrôles Pathologiques sont déterminés sur 1 même lot de réactifs (STA® Staclot® APC-R 010). Chaque échantillon a fait l'objet de 5 mesures. Un même pool a également été dosé en parallèle pour déterminer le nSR-APC-R.

Le tableau VI ci-dessous indique qu'il existe un bon recouvrement des lots, la valeur cible étant 0,40-0,55 pour un contrôle pathologique.

**Tableau VI** :

| | CRT P : 7349A1D | | | | CRT P : 8005A2D | | | | CRT P : 8005B3D | | | |
|---|---|---|---|---|---|---|---|---|---|---|---|---|
| | -PCa | +PCa | APC-SR | n-APC-SR | -PCa | +PCa | APC-SR | n-AP-SR | -PCa | +PCa | APC-SR | n-APC-SR |
| Pool 08/97 | 36,7 | 161,7 | 4,41 | | | | | | | | | |
| | 39,4 | 163,8 | 4,16 | | | | | | | | | |
| 1 | 41,3 | 85,8 | 2,08 | 0,49 | 40,7 | 70,8 | 1,74 | 0,41 | 39,9 | 70,9 | 1,78 | 0,42 |
| 2 | 42,5 | 86,8 | 2,04 | 0,48 | 39,8 | 70,9 | 1,78 | 0,42 | 39,8 | 71,8 | 1,80 | 0,42 |
| 3 | 42,6 | 85,7 | 2,01 | 0,47 | 40,0 | 70,5 | 1,76 | 0,41 | 40,4 | 71,3 | 1,76 | 0,41 |
| 4 | 42,0 | 85,0 | 2,02 | 0,47 | 39,6 | 69,6 | 1,76 | 0,41 | 40,2 | 71,4 | 1,78 | 0,41 |
| 5 | 42,1 | 85,4 | 2,03 | 0,47 | 40,8 | 72,3 | 1,77 | 0,41 | 39,5 | 70,8 | 1,79 | 0,42 |
| MOY | 42,10 | 85,74 | 2,04 | 0,48 | 40,18 | 70,82 | 1,76 | 0,41 | 39,96 | 71,24 | 1,78 | 0,42 |
| SD | 0,51 | 0,67 | 0,03 | 0,01 | 0,54 | 0,97 | 0,02 | 0,00 | 0,35 | 0,40 | 0,02 | 0,00 |
| %CV | 1,22 | 0,78 | 1,24 | 1,24 | 1,34 | 1,37 | 0,90 | 0,90 | 0,88 | 0,57 | 0,86 | 0,86 |

L'analyse des résultats inter-lots de réactifs STA® Staclot® APC-R regroupés dans le tableau VII suivant montre également un bon recouvrement inter-lots de réactifs.

La déviation maximum d'un lot à l'autre est inférieure à 0,04 (valeur absolue) du nSR-APC-R pour le contrôle pathologique.

## Tableau VII :

| | STA STACLOT APCR 008 | | | | STA STACLOT APCR 009 | | | | STA STACLOT APCR 010 | | | |
|---|---|---|---|---|---|---|---|---|---|---|---|---|
| | -PCa | +PCa | APC-SR | n-APC-SR | -PCa | +PCa | APC-SR | n-APC-SR | -PCa | +PCa | APC-SR | n-APC-SR |
| Pool SD6 002 | 46,5 | 180,9 | 3,89 | | 46,3 | 185,7 | 4,01 | | 42,2 | 168,0 | 3,98 | |
| | 45,9 | 179,6 | 3,91 | | 46,2 | 184,9 | 4,00 | | 42,1 | 167,8 | 3,99 | |
| CRT P : 7349A1D | 45,8 | 86,8 | 1,90 | 0,49 | 44,5 | 89,4 | 2,01 | 0,50 | 41,3 | 82,3 | 1,99 | 0,50 |
| | 47,1 | 87,1 | 1,85 | 0,47 | 44,5 | 88,3 | 1,98 | 0,50 | 42,1 | 83,0 | 1,97 | 0,49 |
| CRT P : 8005A2D | 44,3 | 73,6 | 1,66 | 0,43 | 43,0 | 76,2 | 1,77 | 0,44 | 39,4 | 68,4 | 1,74 | 0,44 |
| | 44,4 | 74,9 | 1,69 | 0,43 | 42,4 | 74,0 | 1,75 | 0,44 | 39,7 | 70,4 | 1,77 | 0,45 |
| CRT P : 8005B3D | 44,5 | 75,9 | 1,71 | 0,44 | 43,4 | 74,6 | 1,72 | 0,43 | 39,7 | 70,3 | 1,77 | 0,44 |
| | 45,4 | 74,5 | 1,64 | 0,42 | 43,0 | 76,5 | 1,78 | 0,44 | 40,7 | 70,2 | 1,72 | 0,43 |

**Exemple 7 : Etude de la stabilité.**

1. Conservation :

• Une étude de stabilité accélérée a été réalisée sur les compositions contrôles pathologiques à 30°C pendant 3 + 2 semaines, en comparaison à ces mêmes contrôles conservés à 2-8°C. L'étude des résultats regroupés dans le tableau VIII montre une bonne conservation des contrôles sous forme lyophilisée (Δ relatif des temps + PCa sur 3 semaines < à 5 %).

EP 0 959 356 B1

2. Stabilité des réactifs :

• On a étudié la stabilité des contrôles après leur première reconstitution à 4 températures :

**Tableau VIII : Conservation des contrôles pathologiques APCr pendant 3, 4, 5 semaines à + 30° C**

**Staclot APCr 010**
**Pool F 08/97**
**STA 502**

| | Contrôles gardés à 4°C | | | | 3 semaines | | | | 4 semaines | | | | 5 semaines | | | |
|---|---|---|---|---|---|---|---|---|---|---|---|---|---|---|---|---|
| | -PCa | +PCa | APC -SR | n-APC -SR | -PCa | +PCa | APC -SR | n-APC -SR | -PCa | +PCa | APC -SR | n-APC -SR | -PCa | +PCa | APC -SR | n-APC -SR |
| 7349A1D | 41,5 | 80,6 | 1,94 | 0,48 | 42,0 | 79,8 | 1,90 | 0,47 | 42,0 | 80,4 | 1,91 | 0,47 | 41,8 | 82,0 | 1,96 | 0,48 |
| 8006A2D | 39,4 | 67,6 | 1,72 | 0,42 | 39,8 | 68,2 | 1,71 | 0,42 | 39,2 | 68,6 | 1,75 | 0,43 | 39,2 | 68,8 | 1,76 | 0,43 |
| 8006B3D | 40,2 | 69,4 | 1,73 | 0,42 | 39,7 | 69,2 | 1,74 | 0,43 | 39,9 | 69,9 | 1,75 | 0,43 | 39,7 | 70,8 | 1,78 | 0,44 |

| | Delta relatif (T0/T3 sem.) | | | | Delta relatif (T0/T4 sem.) | | | | Delta relatif (T0/T5 sem.) | | | |
|---|---|---|---|---|---|---|---|---|---|---|---|---|
| | -PCa | +PCa | APC -SR | n-APC -SR | -PCa | +PCa | APC -SR | n-APC -SR | -PCa | +PCa | APC -SR | n-APC -SR |
| 7349A1D | 1,1 | -0,9 | -2,0 | -2,0 | 1,2 | -0,2 | -1,4 | -1,4 | 0,7 | 1,7 | 1,0 | 1,0 |
| 8006A2D | 1,1 | -1,0 | 0,2 | 0,2 | -0,4 | 1,5 | 1,9 | 1,9 | -0,5 | 1,9 | 2,4 | 2,4 |
| 8026B3D | -1,1 | -0,2 | 0,9 | 0,9 | -0,6 | 0,7 | 1,4 | 1,4 | -1,1 | 2,0 | 3,2 | 3,2 |

| | -PCa | +PCa | APC -SR |
|---|---|---|---|
| Pool 08/97 | 37,3 | 158.3 | 4,24 |
| | 40,2 | 157.2 | 3,91 |

13

- 2-8°C, flacons bouchés (étude de 3 lots),
- 25°C, flacons bouchés (étude de 3 lots) placés à l'étuve,
- 15-19°C sur STA, flacons ouverts (étude de 3 lots).
- - 20°C, flacons bouchés.

• Toutes les déterminations des temps de coagulation ont été réalisées sur STA en duplicata sur un même lot de réactifs (Staclot APC-R (010)) et par rapport au même pool aliquoté. L'analyse des données regroupées dans les tableaux IX, X, XI et XII permet de proposer comme stabilité :

- stabilité dans l'appareil : 8h
- stabilité à 2-8°C : 8h
- stabilité à 25°C : 6h.
- stabilité à - 20° C : > 24h.

EP 0 959 356 B1

### TABLEAU IX : Stabilité des contrôles P APCr à 2-8° C (flacons fermés)

**Staclot APCR 010**

| | T = 0 | | | | T = 4 heures | | | | T = 8 heures | | | | T = 12 heures | | | |
|---|---|---|---|---|---|---|---|---|---|---|---|---|---|---|---|---|
| | -PCa | +PCa | APC -SR | n-APC -SR | -PCa | +PCa | APC -SR | n-APC -SR | -PCa | +PCa | APC -SR | n-APC -SR | -PCa | +PCa | APC -SR | n-APC -SR |
| Pool 08/97 | 39,6 | 163,3 | 4,12 | | | | | | | | | | | | | |
| 7349A1D | 40,1 | 82,3 | 2,05 | 0,50 | 41,0 | 84,6 | 2,06 | 0,50 | 41,0 | 84,4 | 2,06 | 0,50 | 42,5 | 86,7 | 2,04 | 0,50 |
| 8005B3D | 39,2 | 72,2 | 1,84 | 0,45 | 39,6 | 72,9 | 1,84 | 0,45 | 39,9 | 74,7 | 1,87 | 0,45 | 40,6 | 76,4 | 1,88 | 0,46 |
| 8026A4D | 37,5 | 64,0 | 1,71 | 0,41 | 37,6 | 64,8 | 1,72 | 0,42 | 38,9 | 65,5 | 1,69 | 0,41 | 38,7 | 66,9 | 1,73 | 0,42 |

| | T = 20 heures | | | | T = 24 heures | | | | Delta relatif % (T0/T8) | | | | Delta relatif % (T0/12) | | | |
|---|---|---|---|---|---|---|---|---|---|---|---|---|---|---|---|---|
| | -PCa | +PCa | APC -SR | n-APC -SR | -PCa | +PCa | APC -SR | n-APC -SR | -PCa | +PCa | APC -SR | n-APC -SR | -PCa | +PCa | APC -SR | n-APC -SR |
| 7349A1D | 42,5 | 89,4 | 2,10 | 0,51 | 43,3 | 89,6 | 2,07 | 0,50 | 2,2 | 2,6 | 0,4 | 0,4 | 6,1 | 5,4 | -0,7 | -0,7 |
| 8005B3D | 42,0 | 79,4 | 1,89 | 0,46 | 41,5 | 79,5 | 1,92 | 0,47 | 1,8 | 3,4 | 1,6 | 1,6 | 3,4 | 5,8 | 2,3 | 2,3 |
| 8026A4D | 39,7 | 70,1 | 1,77 | 0,43 | 40,8 | 72,8 | 1,78 | 0,43 | 3,6 | 2,3 | -1,2 | -1,2 | 3,1 | 4,5 | 1,3 | 1,3 |

EP 0 959 356 B1

## TABLEAU X : Stabilité des contrôles P APCr à 25° C (flacons fermés)

**Staclot APCR 010**

| | T = 0 | | | | T = 2 heures | | | | T = 4 heures | | | |
|---|---|---|---|---|---|---|---|---|---|---|---|---|
| | -PCa | +PCa | APC-SR | n-APC-SR | -PCa | +PCa | APC-SR | n-APC-SR | -PCa | +PCa | APC-SR | n-APC-SR |
| Pool 08/97 | 40,2 | 156,9 | 3,90 | | | | | | | | | |
| 7349A1D | 41,5 | 83,0 | 2,00 | 0.51 | 41,1 | 81,3 | 1,98 | 0,51 | 41,0 | 82,4 | 2,01 | 0,51 |
| 8005B3D | 38,8 | 69,5 | 1,79 | 0,46 | 39,1 | 70,9 | 1,81 | 0,46 | 39,2 | 70,5 | 1,80 | 0,46 |
| 8026A4D | 37,9 | 64,2 | 1,69 | 0,43 | 37,7 | 63,4 | 1,68 | 0,43 | 37,5 | 63,6 | 1,70 | 0,43 |

| | T = 6 heures | | | | T = 8 heures | | | | Delta relatif % (T0/T8) | | | |
|---|---|---|---|---|---|---|---|---|---|---|---|---|
| | -PCa | +PCa | APC-SR | n-APC-SR | -PCa | +PCa | APC-SR | n-APC-SR | -PCa | +PCa | APC-SR | n-APC-SR |
| 7349A1D | 41,3 | 82,7 | 2,00 | 0.51 | 41,4 | 83,1 | 2,01 | 0,51 | -0,2 | 0,1 | 0,4 | 0,4 |
| 8005B3D | 39,1 | 70,6 | 1,80 | 0,46 | 39,9 | 71,0 | 1,78 | 0,46 | 2,8 | 2,2 | -0,6 | -0,6 |
| 8026A4D | 38,1 | 63,9 | 1,68 | 0,43 | 37,8 | 63,5 | 1,68 | 0,43 | -0,3 | -1,0 | -0,8 | -0,8 |

### TABLEAU XI : Stabilité des contrôles P APCr on board (dans l'appareil)

**STA Staclot APCR 010**

| | T = 0 | | | | T = 2 heures | | | | T = 4 heures | | | | T = 6 heures | | | |
|---|---|---|---|---|---|---|---|---|---|---|---|---|---|---|---|---|
| | -PCa | +PCa | APC-SR | n-APC-SR | -PCa | +PCa | APC-SR | n-APC-SR | -PCa | +PCa | APC-SR | n-APC-SR | -PCa | +PCa | APC-SR | n-APC-SR |
| Pool 08/97 | 39,1 | 158,5 | 4,05 | | | | | | | | | | | | | |
| 7349A1D | 41,8 | 82,1 | 1,97 | 0,49 | 42,7 | 84,4 | 1,98 | 0,49 | 42,0 | 83,1 | 1,98 | 0,49 | 42,3 | 82,1 | 1,94 | 0,48 |
| 8005B3D | 40,0 | 70,9 | 1,77 | 0,44 | 40,8 | 71,2 | 1,75 | 0,43 | 40,5 | 71,3 | 1,76 | 0,43 | 40,2 | 72,2 | 1,80 | 0,44 |
| 8026A4D | 37,1 | 62,9 | 1,69 | 0,42 | 37,8 | 64,2 | 1,70 | 0,42 | 37,8 | 64,8 | 1,72 | 0,42 | 38,1 | 63,6 | 1,67 | 0,41 |

| | T = 8 heures | | | | T = 10 heures | | | | Delta relatif % (T0/T8) | | | | Delta relatif % (T0/10) | | | |
|---|---|---|---|---|---|---|---|---|---|---|---|---|---|---|---|---|
| | -PCa | +PCa | APC-SR | n-APC-SR | -PCa | +PCa | APC-SR | n-APC-SR | -PCa | +PCa | APC-SR | n-APC-SR | -PCa | +PCa | APC-SR | n-APC-SR |
| 7349A1D | 41,6 | 82,7 | 1,99 | 0,49 | 42,1 | 83,7 | 1,99 | 0,49 | -0,4 | 0,7 | 1,0 | 1,0 | 0,7 | 1,9 | 1,2 | 1,2 |
| 8005B3D | 40,0 | 71,9 | 1,80 | 0,44 | 39,8 | 71,5 | 1.80 | 0,44 | 0,1 | 1,5 | 1,4 | 1,4 | -0,5 | 0,9 | 1,4 | 1,4 |
| 8026A4D | 37,8 | 64,4 | 1,71 | 0,42 | 37,9 | 64,3 | 1,70 | 0,42 | 1,8 | 2,5 | 0,7 | 0,7 | 2,0 | 2,2 | 0,2 | 0,2 |

EP 0 959 356 B1

## TABLEAU XII : Stabilité des contrôles pathologiques APCr à -20°C

### STA STACLOT APCR 010

| | Contrôles "frais" | | | | Contrôles gardés 24h à -20°C | | | | Delta relatif % | | | |
|---|---|---|---|---|---|---|---|---|---|---|---|---|
| | -PCa | +PCa | APC -SR | n-APC -SR | -PCa | +Pca | APC -SR | n-APC -SR | -PCa | +PCa | APC -SR | n-APC -SR |
| Pool 08/97 | 40,1 | 163,0 | 4,06 | | | | | | | | | |
| CRT P : 7349A1D | 41,1 | 81,3 | 1,98 | 0,49 | 41,3 | 83,4 | 2,02 | 0,50 | -0,5 | -2,6 | -2,1 | -2,1 |
| CRT P : 8005B3D | 39,8 | 71,2 | 1,79 | 0,44 | 39,4 | 71,1 | 1,81 | 0,44 | 1,1 | 0,1 | -1,0 | -1,0 |
| CRT P : 8026A4D | 38,0 | 62,2 | 1,69 | 0,42 | 37,8 | 63,4 | 1,68 | 0,41 | 0,5 | 1,3 | 0,8 | 0,8 |

EP 0 959 356 B1

**Exemple de kit contenant à titre de contrôle pathologique des compositions selon l'invention.**

**Composition :**

- Chaque coffret contient 8 flacons (4 normaux et 4 pathologiques) de plasma citraté lyophilisé (1 ml après reconstitution).

**Exemple de valeur :**

Chaque coffret de Contrôles Normal et Pathologique APC-R contient un papillon qui renferme les informations suivantes : n° de lot, référence du coffret, référence du réactif, date de péremption, valeur déterminée sur STA® du temps de coagulation de ces réactifs dans le système STA® Staclot® APC-R.

Ces temps peuvent varier d'un lot à l'autre mais sont indiqués précisément pour chaque lot (voir papillon inclus dans le coffret).

**Préparation du réactif :**

Agiter très exactement 1 ml d'eau distillée dans chaque flacon. Agiter vigoureusement. Laisser la solution se stabiliser pendant 1 heure à température ambiante (18-25°C). Agiter doucement pour homogénéiser avant emploi.

**Stabilité des réactifs :**

- Lyophilisation à 2-8°C pour une conservation possible jusqu'à la date de péremption indiquée sur le coffret.
- Reconstitution avec de l'eau distillée sachant que la stabilité des lots est la suivante :

  - 8h sur STA® (15-19°C)
  - 8h à 2-8°C
  - 6h à 25°C.

**Mode d'emploi :**

Les plasmas contrôles sont utilisés de façon identique à celle des plasmas à tester.

**REFERENCES**

- AMER L. et al., *Thromb. Res.*, 1990, 57, pages 247-258,
- BERTINA R.M. et al., *Nature*, 1994, 369, pages 64-68,
- DAHLBÄCK B. et al., *Thromb. Haemost.*, 1991, 65, abstract 39, page 658.
- DAHLBÄCH B. et al., *Proc. Natl. Acad. Sci*. USA, 1993, 90, pages 1004-1008
- DAHLBÄCK et al., *Proc. Natl. Acad. Sci*. USA, 1994, 91, pages 1396-1400.
- DE RONDE H. et al., *Thromb. Haemost.*, 1994, 72, pages 880-886,
- KALAFATIS M.et al., *J. Biol. Chem*., 1994, 269, pages 31869-31880,
- KARGES H.E. et al., Arzneim.-Forsch. / *Drug Res., 44 (1), Nr. 6 (1994), P. 793-797)*.
- LEWIS J.H. : *Comparative Hemostasis in vertebrates*, 1996, page 339
- MITCHELL C.A. et al., *N. Engl. J. Med.*, 1987, 317, pages 1638-1642,
- SOULIER J.P., *NRFH, 1975, tome 15, N° 2, pp 195-212*
- SVENSSON P.J. et al., *N. Engl. J. Med.*, 1994, 330, pages 517-522,
- VARADI K. et al., *Thromb. Haemost.*, 1995, 73, pages 730-731

**Revendications**

1. Utilisation d'un sérum de bovin riche en facteur Va pour la préparation de compositions utiles en tant que contrôles pathologiques ou calibrants dans une méthode de détection chez l'homme d'une résistance à la protéine C activée (PCa).

2. Utilisation selon la revendication 1 **caractérisée en ce que** le sérum est préalablement traité de façon à éliminer les effets de facteurs dépendants de la vitamine K.

3. Utilisation selon la revendication 1 ou 2 **caractérisée en ce que** le sérum est préalablement traité de façon à éliminer les facteurs II, VII, IX, X, protéine S, protéine C et protéine Z, ainsi que les traces de fibrine.

4. Utilisation selon l'une des revendications 1 à 3 **caractérisée en ce que** le sérum est préalablement adsorbé sur du sulfate de baryum et de la bentonite.

5. Utilisation selon l'une des revendications 1 à 4 **caractérisée en ce que** le sérum contient une quantité de facteur Va supérieure à 20 U/ml (2000 %).

6. Utilisation selon la revendication 5 **caractérisée en ce que** le sérum contient une quantité de facteur Va supérieure à 22 U/ml (2200%).

7. Composition utile en tant que contrôle pathologique dans une méthode de détection d'une résistance à la protéine C activée comprenant du plasma normal humain et du sérum de bovin riche en facteur Va.

8. Composition selon la revendication 7 **caractérisée en ce que** le plasma normal humain est du plasma humain citraté.

9. Composition selon l'une des revendications 7 et 8 **caractérisée en ce que** le sérum est préalablement traité de façon à éliminer les effets de facteurs dépendants de la vitamine K.

10. Composition selon l'une des revendications 7 et 8 **caractérisée en ce que** le sérum est préalablement traité de façon à éliminer les facteurs II, VII, IX, X, protéine S, protéine C et protéine Z ainsi que les traces de fibrine.

11. Composition selon l'une des revendications 7 à 10 **caractérisée en ce que** le sérum est préalablement absorbé sur du sulfate de baryum et de la bentonite.

12. Composition selon l'une des revendications 7 à 11 **caractérisée en ce que** le sérum contient une quantité de facteur Va supérieure à 20 U/ml (2000 %).

13. Composition selon la revendication 12 **caractérisée en ce que** le sérum contient une quantité de facteur Va supérieure à 22 U/ml (2200 %).

14. Composition selon l'une des revendications 7 à 13 **caractérisée en ce que** la dilution finale du sérum dans le plasma normal humain est située dans une gamme comprise entre 1/300 et 1/1200.

15. Composition selon la revendication 14 **caractérisée en ce que** la dilution finale du sérum dans le plasma normal humain est comprise entre 1/400 et 1 /1000.

16. Procédé de préparation de compositions selon la revendication 7 **caractérisé en ce que** l'on effectue une gamme de dilutions d'un sérum de bovin riche en facteur Va dans un plasma normal humain et l'on sélectionne les compositions pour lesquelles le temps de coagulation en présence de PCa se situe dans l'intervalle défini pour le temps de coagulation en présence de PCa d'un plasma présentant une APC-R/une résistance à la PCa.

17. Procédé selon la revendication 16 **caractérisé en ce que** le plasma normal humain est du plasma humain citraté.

18. Procédé selon la revendication 16 ou 17 **caractérisé en ce que** le sérum de bovin contient plus de 20 U/ml de facteur Va (2000 %).

19. Procédé selon la revendication 18 **caractérisé en ce que** le sérum de bovin contient plus de 22 U/ml de facteur Va (2200 %).

20. Procédé selon l'une des revendications 16 à 19 **caractérisé en ce que** le sérum est préalablement absorbé sur du sulfate de baryum et de la bentonite.

21. Utilisation d'une composition selon la revendication 7 dans une méthode de détection in vitro chez l'homme d'une résistance à la protéine C activée.

22. Utilisation selon la revendication 21 **caractérisée en ce qu'**il s'agit d'une méthode chronométrique dans laquelle les résultats sont préférentiellement exprimés sous forme de rapport normalisé, ou, préférentiellement en temps ou DO.

23. Méthode de détection d'une résistance à la PCa consistant à comparer le temps de coagulation d'un plasma à tester avec celui de compositions selon l'une des revendications 7 à 15 et à évaluer le niveau de résistance de la PCa présenté par ledit plasma.

24. Kit de diagnostic d'une résistance à la protéine C activée, comprenant à titre de contrôle pathologique au moins une composition selon la revendication 7.

**Patentansprüche**

1. Verwendung eines an Faktor Va reichen Rinderserums zur Herstellung von Zusammensetzungen, die als pathologische oder zur Kalibrierung dienende Kontrollen in einem Verfahren zum Nachweis einer Resistenz gegen das aktivierte Protein C (PC-a) beim Menschen nützlich sind.

2. Verwendung nach Anspruch 1, **dadurch gekennzeichnet, dass** das Serum vorab derart behandelt wird, dass die Wirkungen von von Vitamin K abhängigen Faktoren beseitigt werden.

3. Verwendung nach Anspruch 1 oder 2, **dadurch gekennzeichnet, dass** das Serum vorab derart behandelt wird, dass die Faktoren II, VII, IX, X, Protein S, Protein C und Protein Z sowie die Spuren von Fibrin beseitigt werden.

4. Verwendung nach einem der Ansprüche 1 bis 3, **dadurch gekennzeichnet, dass** das Serum vorab an Bariumsulfat und Bentonit adsorbiert wird.

5. Verwendung nach einem der Ansprüche 1 bis 4, **dadurch gekennzeichnet, dass** das Serum eine Menge von Faktor Va über 20 U/ml (2000%) enthält.

6. Verwendung nach Anspruch 5, **dadurch gekennzeichnet, dass** das Serum eine Menge von Faktor Va über 22 U/ml (2200%) enthält.

7. Zusammensetzung, welche als pathologische Kontrolle in einem Verfahren zum Nachweis einer Resistenz gegen das aktivierte Protein C nützlich ist, welche normales menschliches Plasma und an Faktor Va reiches Rinderserum umfasst.

8. Zusammensetzung nach Anspruch 7, **dadurch gekennzeichnet, dass** das normale menschliche Plasma menschliches Citratplasma ist.

9. Zusammensetzung nach einem der Ansprüche 7 und 8, **dadurch gekennzeichnet, dass** das Serum vorab derart behandelt wird, dass die Wirkungen von von Vitamin K abhängigen Faktoren beseitigt werden.

10. Zusammensetzung nach einem der Ansprüche 7 und 8, **dadurch gekennzeichnet, dass** das Serum vorab derart behandelt wird, dass die Faktoren II, VII, IX, X, Protein S, Protein C und Protein Z sowie die Spuren von Fibrin beseitigt werden.

11. Zusammensetzung nach einem der Ansprüche 7 bis 10, **dadurch gekennzeichnet, dass** das Serum vorab an Bariumsulfat und Bentonit adsorbiert wird.

12. Zusammensetzung nach einem der Ansprüche 7 bis 11, **dadurch gekennzeichnet, dass** das Serum eine Menge an Faktor Va über 20 U/ml (2000%) enthält.

13. Zusammensetzung nach Anspruch 12, **dadurch gekennzeichnet, dass** das Serum eine Menge an Faktor Va über 22 U/ml (2200%) enthält.

14. Zusammensetzung nach einem der Ansprüche 7 bis 13, **dadurch gekennzeichnet, dass** die endgültige Verdünnung des Serums in dem normalen menschlichen Plasma in einem Bereich zwischen 1/300 und 1/1200 liegt.

15. Zusammensetzung nach Anspruch 14, **dadurch gekennzeichnet, dass** die endgültige Verdünnung des Serums in dem normalen menschlichen Plasma zwischen 1/400 und 1/1000 liegt.

16. Verfahren zur Herstellung von Zusammensetzungen nach Anspruch 7, **dadurch gekennzeichnet, dass** man eine Reihe von Verdünnungen eines an Faktor Va reichen Rinderserums in einem normalen menschlichen Plasma erzeugt und man die Zusammensetzungen auswählt, bei denen die Gerinnungszeit in Gegenwart von PCa in dem für die Gerinnungszeit eines Plasmas, welches eine APC-R/eine Resistenz gegen PCa zeigt, in Gegenwart von PCa definierten Intervall liegt.

17. Verfahren nach Anspruch 16, **dadurch gekennzeichnet, dass** das normale menschliche Plasma menschliches Citratplasma ist.

18. Verfahren nach Anspruch 16 oder 17, **dadurch gekennzeichnet, dass** das Rinderserum mehr als 20 U/ml Faktor Va (2000%) enthält.

19. Verfahren nach Anspruch 18, **dadurch gekennzeichnet, dass** das Rinderserum mehr als 22 U/ml Faktor Va (2200%) enthält.

20. Verfahren nach einem der Ansprüche 16 bis 19, **dadurch gekennzeichnet, dass** das Serum vorab an Bariumsulfat und Bentonit adsorbiert wird.

21. Verwendung einer Zusammensetzung nach Anspruch 7 in einem in vitro erfolgenden Verfahren zum Nachweis einer Resistenz gegen das aktivierte Protein C beim Menschen.

22. Verwendung nach Anspruch 21, **dadurch gekennzeichnet, dass** es sich um eine zeitmessende Methode handelt, bei welcher die Ergebnisse bevorzugt in Form eines normalisierten Verhältnisses oder vorzugsweise als Zeit oder OD ausgedrückt werden.

23. Verfahren zum Nachweis einer Resistenz gegen PCa, welches darin besteht, die Gerinnungszeit eines zu untersuchenden Plasmas mit jener von Zusammensetzungen nach einem der Ansprüche 7 bis 15 zu vergleichen und das Niveau der PCa-Resistenz, die von dem Plasma gezeigt wird, auszuwerten.

24. Kit zur Diagnose einer Resistenz gegen das aktivierte Protein C, welcher als pathologische Kontrolle wenigstens eine Zusammensetzung nach Anspruch 7 umfasst.

**Claims**

1. Use of a bovine serum rich in factor Va for the preparation of compositions useful as pathological controls or calibrants in a method for the detection in man of resistance to activated protein C (aPC).

2. Use according to Claim 1, **characterized in that** the serum is treated beforehand so as to eliminate the effects of vitamin K-dependent factors.

3. Use according to Claim 1 or 2, **characterized in that** the serum is treated beforehand so as to eliminate factors II, VII, IX, X, protein S, protein C and protein Z, as well as traces of fibrin.

4. Use according to one of Claims 1 to 3, **characterized in that** the serum is adsorbed beforehand on barium sulphate and bentonite.

5. Use according to one of Claims 1 to 4, **characterized in that** the serum contains a quantity of factor Va greater than 20 U/ml (2000%).

6. Use according to Claim 5, **characterized in that** the serum contains a quantity of factor Va greater than 22 U/ml (2200%).

7. Composition useful as a pathological control in a method for the detection of resistance to activated protein C comprising normal human plasma and bovine serum rich in factor Va.

8. Composition according to Claim 7, **characterized in that** the normal human plasma is citrated human plasma.

9. Composition according to one of Claims 7 and 8, **characterized in that** the serum is treated beforehand so as to eliminate the effects of vitamin K-dependent factors.

10. Composition according to one of Claims 7 and 8, **characterized in that** the serum is treated beforehand so as to eliminate factors II, VII, IX, X, protein S, protein C and protein Z, as well as traces of fibrin.

11. Composition according to one of Claims 7 to 10, **characterized in that** the serum is adsorbed beforehand on barium sulphate and bentonite.

12. Composition according to one of Claims 7 to 11, **characterized in that** the serum contains a quantity of factor Va greater than 20 U/ml (2000%).

13. Composition according to Claim 12, **characterized in that** the serum contains a quantity of factor Va greater than 22 U/ml (2200%).

14. Composition according to one of Claims 7 to 13, **characterized in that** the final dilution of the serum in normal human plasma is situated in a range of between 1/300 and 1/1200.

15. Composition according to Claim 14, **characterized in that** the final dilution of the serum in normal human plasma is between 1/400 and 1/1000.

16. Method for the preparation of compositions according to Claim 7, **characterized in that** a range of dilutions of a bovine serum rich in factor Va is carried out in a normal human plasma and the compositions for which the clotting time in the presence of aPC is situated in the interval defined for the clotting time in the presence of aPC of a plasma having aPC-R/aPC-resistance, are selected.

17. Method according to Claim 16, **characterized in that** the normal human plasma is citrated human plasma.

18. Method according to Claim 16 or 17, **characterized in that** the bovine serum contains more than 20 U/ml of factor Va (2000%).

19. Method according to Claim 18, **characterized in that** the bovine serum contains more than 22 U/ml of factor Va (2200%).

20. Method according to one of Claims 16 to 19, **characterized in that** the serum is adsorbed beforehand on barium sulphate and bentonite.

21. Use of a composition according to claim 7, in a method for the in vitro detection in man of resistance to activated protein C.

22. Use according to Claim 21, **characterized in that** it involves a chronometric method in which the results are preferably expressed in the form of a normalized ratio, or preferably as time or OD.

23. Method for the detection of resistance to aPC consisting in comparing the clotting time of a plasma to be tested with that of compositions according to one of Claims 7 to 15 and in evaluating the level of aPC resistance exhibited by said plasma.

**24.** Kit for the diagnosis of resistance to activated protein C, comprising, as pathological control, at least one composition according to Claim 7.